# EUROPEAN PATENT APPLICATION

(11) **EP 4 368 031 A1**
(43) Date of publication of application: **15.05.2024**
(21) Application number: 22837670.3
(22) Date of filing: 05.07.2022
(51) Int. Cl.: A23K 20/20, A23K 20/163, A23K 50/75, A61K 9/28, A61K 31/715, A61K 31/717, A61K 31/719, A61K 31/723, A61K 31/731, A61K 31/732, A61K 31/734, A61K 33/14, A61K 33/26, A61K 47/44, A61P 1/04, A61P 31/04

(54) **CLOSTRIDIUM PERFRINGENS SPORULATION INHIBITOR**

(30) Priority: 07.07.2021 JP 2021112772
(71) Applicant: AJINOMOTO CO., INC., Chuo-ku Tokyo 104-8315 (JP)
(72) Inventor: UEHARA, Akinori, Kawasaki-shi, Kanagawa 210-8681 (JP); SAKAMOTO, Yasuteru, Kawasaki-shi, Kanagawa 210-8681 (JP)
(74) Representative: Strehl Schübel-Hopf & Partner
(86) International application number: PCT/JP2022/026692
(87) International publication number: WO 2023/282255

(57) **Abstract**

The present invention provides a sporulation inhibitor of Clostridium perfringens containing at least one inorganic compound at a concentration that allows Clostridium perfringens to settle in vivo, wherein the inorganic compound is at least one salt selected from the group consisting of K, Na, Mg, Ca, and Fe, and the inorganic compound is coated with a protective layer containing hydrogenated vegetable oil.

## Description

### Technical Field

The present invention relates to a Clostridium perfringens sporulation inhibitor.

### Background Art

Necrotic enteritis (NE) in the poultry industry is an important disease that causes large economic losses (2 billion US$/year) (Non Patent Literature 1). Necrotic enteritis significantly reduces the growth of broilers and leads to death in the worst case. In NE, the mucosa of the small intestine becomes necrotic, forming a yellow-brown or bile-colored pseudomembrane, and a pit-like ulcer on the surface of the mucous membrane in subclinical NE (Non Patent Literature 2).

Necrotic enteritis is known to be caused by Clostridium perfringens, which is a gram-positive bacterium. C. perfringens is known to be a resident bacterium in the intestinal tract of poultry (Non Patent Literature 3). The vegetative cells of C. perfringens are bacilli (1 × 10 µm), but when the environment is adverse, they form spores (cocci with a diameter of 1 µm). It is known that NetB toxin (such as NetB) is secreted during spore germination, and this causes enteritis (Non Patent Literature 4, Figure 1). Accordingly, it is necessary to understand the life cycle of C. perfringens to control the toxin production of C. perfringens.

The C. perfringens is classified into types depending on the type of toxin it produces (Non Patent Literature 5). In 2008, two new types were defined from type A: C. perfringens that produces a toxin called NetB (Necrotic Enteritis Toxin B-like) is defined as type F, and C. perfringens that produces a toxin called CPE (Clostridium Perfringens Enterotoxin) is defined as type G. NetB is reported to be the main factor of NE (Non Patent Literature 6).

Further, it is known that C. perfringens does not have flagella, but it moves by stretching and contracting pili (Type-IV-pillar: T4P) and adheres to the intestinal tract or the like of the host animal (Non Patent Literature 7). Also, the structure and mechanism of T4P pili in Gram-positive bacteria including C. perfringens have been reported, and it is known that the tip of T4P is stretched and contracted by using the ATP energy (Non Patent Literature 8, Figure 1).

There are also reports on the mechanism of action of the Genus Clostridium in the intestinal tract. The Genus Clostridium secretes mucin-degrading enzymes, degrades mucin in the mucus layer, adheres to epithelial cells from thinned areas, induces cell death, and induces inflammation by destroying tight junctions (Non Patent Literature 9, Figure 1).

One method for controlling necrotic enteritis in poultry is to use anti-biotic growth promoters (AGPs). As AGPs, bacitracin methylenedisalicylic acid and Avilamycin are used in poultry (Non Patent Literature 10,11). However, due to problems such as the environmental burden and the emergence of drug-resistant bacteria, restrictions on the use of AGPs have been tightened around the world, they were completely banned in Europe in 2006, and the use of antibiotics such as Tylosin and Colistin, which are commonly used to humans and animals, is prohibited also in the United States. There is a need for an environmentally friendly alternative to AGPs that solves these problems.

As a method without using antibiotics, there is a method using nano-iron oxide as a sporulation inhibitor of Clostridium difficile (Patent Literature 1). The method disclosed herein is a method in which nano-iron oxide inhibits germination by directly acting on Clostridium difficile spores, but the literature does not mention the effect on vegetative cells of C. difficile.

Clostridium difficile described in Patent Literature 1 is different from Clostridium perfringens in terms of phenotype, chemotaxonomy, and phylogenetics, and due to the presence of flagella, it was named Clostridioides difficile in 2016 (Non Patent Literature 12).

### Citation List

### Patent Literature

Patent Literature 1: Japanese Patent No. 6385573

### Non Patent Literature

Non Patent Literature 1: Poult. Sci. (2019) vol. 98, 128-135
Non Patent Literature 2: Poult. Sci. (1992) vol. 71, pp 1145-1153
Non Patent Literature 3: Avian Diseases (2001) vol. 45, pp 887-896
Non Patent Literature 4: Microbiology and Molecular Biology Reviews (2015) vol. 79, No. 1, pp 19-37
Non Patent Literature 5: Anaerobe (2018) vol. 53, pp 5-10
Non Patent Literature 6: PloS Pathogens (2008) vol. 4, Issue 2, e26
Non Patent Literature 7: Trends in Microbiology (2020) vol. 28 (5), pp 340-348
Non Patent Literature 8: Microbiology and Molecular Biology Reviews (2013) vol. 77, No. 3, pp 323-341
Non Patent Literature 9: The Journal of the Japanese Society of Internal Medicine (2016) vol. 106 No. 3, pp 466-471
Non Patent Literature 10: Poult. Sci. (2003) vol. 82, pp 360-363
Non Patent Literature 11: Avian Patho. (2016) vol. 45, No.3, pp 365-369
Non Patent Literature 12: Anaerobe (2016), v 40, pp 95-99

### Summary of Invention

### Technical Problem

Accordingly, it is an object of the present invention to provide an alternative to antibiotics capable of preventing or treating Clostridium perfringens infection such as necrotic enteritis. The present invention also aims to provide a supplement and a feed containing such an alternative. The present invention also aims to provide a method for administering such an alternative.

### Solution to Problem

The inventors have found that when birds are fed with an inorganic compound capable of allowing C. perfringens to settle in the state of vegetative cells, sporulation can be inhibited. In this method, C. perfringens can be excreted from the body as feces before producing a toxin, and therefore Clostridium perfringens infection can be suppressed. Since C. perfringens is resident bacteria also in the human intestinal tract, the aforementioned inorganic compound can be used to prevent or treat Clostridium perfringens infection not only in animals such as birds but also in humans, or as a supplement to alleviate the symptoms of infection. That is, the present application provides the respective inventions below.
1. A sporulation inhibitor of Clostridium perfringens comprising at least one inorganic compound at a concentration that allows Clostridium perfringens to settle in vivo, wherein the inorganic compound is at least one salt selected from the group consisting of K, Na, Mg, Ca, and Fe, and the inorganic compound is coated with a protective layer containing a hydrogenated vegetable oil.
2. The sporulation inhibitor according to 1 above, wherein the inorganic compound is a salt with an anion formed from an acid selected from the group consisting of hydrochloric acid, sulfuric acid, phosphoric acid, carbonic acid, lignosulfonic acid, silicic acid, lactic acid, citric acid, gluconic acid, succinic acid, fumaric acid, iodine, and iodic acid.
3. The sporulation inhibitor according to 1 or 2 above, wherein the inorganic compound is at least one selected from the group consisting of KCl, NaCl, MgCl₂, CaCl₂, and FeCl₂.
4. The sporulation inhibitor according to any one of 1 to 3 above, wherein the inorganic compound is KCl.
5. The sporulation inhibitor according to any one of 1 to 4 above, further comprising a polysaccharide.
6. The sporulation inhibitor according to 5 above, wherein the polysaccharide is at least one selected from the group consisting of pullulan, xanthan gum, guar gum, carrageenan, arabic gum, pectin, carboxymethylcellulose, chondroitin, tara gum, locust bean gum, alginates (such as sodium salt, potassium salt, calcium salt, or ammonium salt), alginic acid ester, and a mixture thereof.
7. The sporulation inhibitor according to 5 above, wherein the polysaccharide is arabic gum.
8. The sporulation inhibitor according to any one of 1 to 7 above, wherein the inorganic compound and the polysaccharide contained as needed are coated with a protective layer containing a hydrogenated vegetable oil.
9. The sporulation inhibitor according to 8 above, wherein the hydrogenated vegetable oil is a hydrogenated oil of rapeseed oil, linseed oil, safflower oil, sunflower oil, soybean oil, corn oil, peanut oil, cottonseed oil, sesame oil, rice oil, olive oil, palm oil, palm kernel oil, or coconut oil.
10. The sporulation inhibitor according to any one of 1 to 7 above, wherein the inorganic compound and the polysaccharide contained as needed are protected by two layers of hydrogenated rapeseed oil and benzoic acid resin.
11. A supplement to prevent or treat Clostridium perfringens infection, comprising the sporulation inhibitor according to any one of 1 to 10 above.
12. The supplement according to 10 or 11 above, comprising an inorganic compound at a concentration that allows administration of the inorganic compound at a dose of 1 mg/kg body weight/day or more.
13. The supplement according to 10 above, wherein the Clostridium perfringens infection is necrotic enteritis.
14. A feed comprising the sporulation inhibitor according to any one of 1 to 8 above.
15. The feed according to 14 above, further comprising a conventional feed, wherein the concentration of the inorganic compound is 0.013 mmol/kg or more with reference to the mass of the conventional feed.
16. A method for preventing or treating Clostridium perfringens infection of nonhuman animals, the method comprising administering an inorganic compound of at least one salt selected from the group consisting of K, Na, Mg, Ca, and Fe to the intestinal tract of a nonhuman animal.
17. A method for inhibiting sporulation of Clostridium perfringens in vitro, the method comprising incubating at an effective concentration of 500 mM or more of an inorganic compound of at least one salt selected from the group consisting of K, Na, Ca, and Fe.

### Advantageous Effects of Invention

According to the present invention, it is possible to prevent vegetative cells of C. perfringens from changing into spores. According to the present invention, it is also possible to prevent or treat necrotic inflammation due to C. perfringens. According to the present invention, it is further possible to exert a weight gain effect of livestock, while preventing or treating Clostridium perfringens infection.

### Brief Description of Drawings

[Figure 1] Figure 1 shows the results of a settling experiment of Clostridium perfringens ATCC10873.
[Figure 2] Figure 2 shows the results of an experiment to observe the agglomeration and cell shapes of three Clostridium perfringens strains (ATCC10873, SM101, and CNEOP004).
[Figure 3] Figure 3 is a phase-contrast microscopic observation image of Clostridium perfringens SM101 spores.
[Figure 4] Figure 4 is a phase-contrast microscopic observation image of Clostridium perfringens SM101 cocci when KCl (500 mM) is added.
[Figure 5] Figure 5 shows the results of an agglomeration experiment of mixed bacterial cells of Clostridium perfringens ATCC 10873, Salmonella enterica IAM1648, and E.coli MG1655KCl when KCl (500 mM) w/1% AG and NaCl (500 mM) w/1% AG are added.
[Figure 6] Figure 6 shows the results of an enteric test for Coated-Arabic Gum.
[Figure 7] Figure 7 shows the results of a dissolution test for Coated-KCl.

### Description of Embodiments

### [Abbreviations]

AG: Arabic Gum
AGPs: Anti-biotic growth promoters (Antibiotics for growth promotion)
BMD: Bacitracin Methylene Disalicylate
BWG: Body Weight Gain (Weight gain effect)
Cp: Clostridium perfringens
DW: Distilled water (Pure water)
NE: Necrotic Enteritis
NT: Non treatment (Non-treated, Additive-free)
OD: Optical Density

### [Inorganic compound]

The inorganic compound to be used in the present invention is at least one salt selected from the group consisting of K, Na, Mg, Ca, and Fe. The anion constituting the salt is not specifically limited, but examples thereof include anion formed from an acid selected from the group consisting of hydrochloric acid, sulfuric acid, phosphoric acid, carbonic acid, lignosulfonic acid, silicic acid, lactic acid, citric acid, gluconic acid, succinic acid, fumaric acid, iodine, and iodic acid. From an economic point of view, Cl⁻ is preferable.

Specifically, the inorganic compound is preferably at least one selected from the group consisting of KCl, NaCl, MgCl₂, CaCl₂, and FeCl₂. More preferably, the inorganic compound contains KCl. The inorganic compound is further preferably KCl.

In the sporulation inhibitor of the present invention, the concentration of the inorganic compound is not specifically limited, as long as the concentration is sufficient to settle Clostridium perfringens in vivo without agglomeration. The concentration required for settling varies depending on the type of the inorganic compound or the concentration of bacterial cells, and the KCl concentration is 100 mM or more, the NaCl concentration is 500 mM or more, the MgCl₂ concentration is over 500 mM, and the FeCl₂ concentration is 10 mM or more, for example, when the concentration of bacterial cells is an optical density of about 2.0 at a wavelength of 660 nm. Although the concentration of the inorganic compound may be increased in order to exert the settling effect, the effect reaches a ceiling at a certain concentration. Therefore, it is desirably 1,000 mM or less, for example, from an economic point of view.

### [Polysaccharide]

It has been reported that polysaccharides exert an agglomeration effect on Gram-negative bacteria (International Publication No. 2019/177172), but experiments conducted by the inventors showed that they did not exert neither settling effect nor agglomeration effect on Clostridium perfringens that is a Gram-positive bacterium (see Examples 1 and 5 described below). However, it was found that when combined with the aforementioned inorganic compound, the inorganic compound enhanced the settling effect and agglomeration effect of C. perfringens. In particular, it was found that when the sporulation inhibitor of the present invention exerts an agglomeration effect on Gram-positive bacteria, it can also improve the weight gain effect of livestock. Accordingly, the sporulation inhibitor of the present invention may further contain a polysaccharide.

Examples of the polysaccharide that can be used in the present invention include at least one selected from the group consisting of pullulan, xanthan gum, guar gum, carrageenan, arabic gum, pectin, carboxymethylcellulose, chondroitin, tara gum, locust bean gum, alginates (such as sodium salt, potassium salt, calcium salt, or ammonium salt), alginic acid ester, and a mixture thereof. Among these, arabic gum, carboxymethylcellulose, guar gum, carrageenan, locust bean gum, and pullulan are preferable, and arabic gum is more preferable, from the point of view of cost effectiveness and feed registration status.

The polysaccharide can be given to the subject together with the inorganic compound or separately. For example, as will be described below, when forming a sporulation inhibitor by applying a coating agent to the inorganic compound and the polysaccharide, a coating agent may be applied to the inorganic compound and the polysaccharide together, and they may be given to a subject as an agent in which the inorganic compound and the polysaccharide coexist in one sporulation inhibitor, or a coating agent may be applied to the inorganic compound to form a sporulation inhibitor, which may be given to a subject together with a coating agent applied to the polysaccharide.

In the sporulation inhibitor of the present invention, the concentration of the polysaccharide can be appropriately determined, but it is preferably 0.5 to 3 mass% from an economic point of view. More preferably, it is 1 to 2 mass%. In this description, unless otherwise specified, the unit "%" represents mass%.

The concentration of the inorganic compound required to settle Clostridium perfringens in vivo can be lower when used in combination with a polysaccharide than when no polysaccharide is used. For example, a settling effect can be exerted with a KCl concentration in the sporulation inhibitor of the present invention containing KCl of 50 mM or more, when used in combination with arabic gum. It can be preferably 100 mM or more (for example, 100 to 1,000 mM), more preferably 200 mM or more (for example, 200 to 700 mM). Although the concentration of the inorganic compound may be increased in order to exert the settling effect, the effect reaches a ceiling at a certain concentration. Therefore, it is desirably 500 mM or less, for example, from an economic point of view.

The sporulation inhibitor of the present invention may contain an excipient. The excipient is not specifically limited, as long as it is commonly used to improve shaping and is pharmacologically acceptable, but examples thereof include calcium carbonate, silicon dioxide, calcium silicate, zeolite, sorbitol, corn starch, talc, yeast bentonite, chaff, liquid paraffin, and polysaccharides, monosaccharides, and disaccharides other than polysaccharides that have the property of agglomerating Clostridium perfringens. When the sporulation inhibitor of the present invention contains an excipient, the amount of the excipient is generally preferably 0.1 to 100 parts by mass with respect to 100 parts by mass of the sporulation inhibitor.

The sporulation inhibitor of the present invention may also contain an optional additive that can be contained in supplements or feeds. Examples of the additive include amino acids, organic acids, vitamins, color enhancers (carotenoids), flavoring agents, and attenuated vaccines. When the sporulation inhibitor contains an optional additive, the amount of the optional additive is generally preferably 0.1 to 100 parts by mass with respect to 100 parts by mass of the sporulation inhibitor.

### [Coating agent]

The coating agent forms a protective layer of the inorganic compound. The coating agent is not particularly limited, as long as it is a substance that can form an enteric coating agent and is safe for ingestion by livestock or humans. The coating agent may be used alone, or two types or more thereof may be used in combination. From the viewpoint of ease of handling and economy, the coating agent is preferably a hydrogenated vegetable oil or a substance that is commonly used as a coating agent for tablets, such as benzoic acid resin, shellac, zein, hydroxypropylmethylcellulose, and maltitol. Examples of the hydrogenated vegetable oil include a hydrogenated oil of rapeseed oil, linseed oil, safflower oil, sunflower oil, soybean oil, corn oil, peanut oil, cottonseed oil, sesame oil, rice oil, olive oil, palm oil, palm kernel oil, or coconut oil. The hydrogenated vegetable oil is preferably a hydrogenated oil of rapeseed oil, linseed oil, safflower oil, sunflower oil, soybean oil, corn oil, peanut oil, cottonseed oil, sesame oil, rice oil, olive oil, palm oil, palm kernel oil, or coconut oil. Among these, hydrogenated rapeseed oil and benzoic acid resin are preferable as the coating agent. A layer of hydrogenated rapeseed oil is preferable since it allows the core to be dissolved in a short time. A layer of benzoic acid resin is preferable since it allows the sporulation inhibitor to be dissolved in neutral to alkaline conditions (after passing through the stomach).

The coating agent is preferably in an amount of 5 to 90 mass%, more preferably 20 to 30 mass%, based on the total mass of the sporulation inhibitor of the present invention. The coating agent may also contain an optional additive that can be contained in feeds or pharmaceuticals for humans.

The coating may be a single layer or multiple layers of two or more layers. A multilayer coating is preferable since it makes easier to control the dissolution rate in the body. In particular, when the outermost layer is a layer of hydrogenated rapeseed oil, and the innermost layer in contact with the inorganic compound is a layer of benzoic acid resin, the coating agent does not dissolve in the stomach but dissolves in the intestines, which is preferable.

The dissolution rate of the sporulation inhibitor of the present invention in gastric juices is desirably less than 60%, and the dissolution rate in intestinal juices is desirably 70% or more. In order to achieve such dissolution rates, it can be adjusted by forming a two-layer film or a multilayer film, or by controlling the type or film thickness of the coating agent of each layer.

It is particularly preferable that the inorganic compound is a K salt (particularly KCl), the polysaccharide is arabic gum, and the coating agent is at least one selected from the group consisting of hydrogenated rapeseed oil and benzoic acid resin. Among these, it is particularly preferable that the inorganic compound is a K salt (particularly KCl), the polysaccharide is arabic gum, and the coating agent is composed of two layers of hydrogenated rapeseed oil and benzoic acid resin. In this case, it is further particularly preferable that the layer formed from the benzoic acid resin is in contact with the inorganic compound, and a layer formed from hydrogenated rapeseed oil is formed further thereon. In particular, it is most preferable that the KCl concentration in the sporulation inhibitor is 500 mM, the arabic gum concentration is 1%, a layer formed from benzoic acid resin is in contact with the inorganic compound, and a layer formed from hydrogenated rapeseed oil is formed further thereon.

### [Coating method]

The method for coating the sporulation inhibitor is not specifically limited, but a coated sporulation inhibitor can be obtained, for example, by spraying a coating agent heated to a temperature higher than the melting point to be in liquid form while fluidizing the powder or granular core using a commercially available fluidized bed spray granulator. The coated sporulation inhibitor preferably has a size of about 0.05 to 5mm, for facilitating handling. Further, the temperature for heating the coating agent is not particularly limited as long as it is equal to or higher than the melting point of the coating agent, but is preferably higher than the melting point of the coating agent by about 5°C to 15°C.

The sporulation inhibitor of the present invention can be orally ingested by humans or nonhuman animals, or can be directly administered to the intestinal tract of a nonhuman animal. When directly administering to the intestinal tract, since there is no need to consider deactivation due to gastric acid or irritation to the gastric mucosa, there is no need to cover with a protective layer containing a hydrogenated vegetable oil. In both cases of oral ingestion and administration to the intestinal tract, the amount and frequency of administration of the inorganic compound to be ingested by the subject are the same as for the supplements described below.

### [Supplement]

The sporulation inhibitor of the present invention can also be used as a supplement for humans or nonhuman animals. The form of the supplement is not particularly limited, and examples thereof can include tablets, granules, powders, and drink agents. The supplement of the present invention may further contain components known as components for supplements such as antioxidants and proteins, in addition to the sporulation inhibitor of the present invention. It is appropriate to take the supplement of the present invention continuously every day. The amount of the supplement of the present invention to be taken varies, for example, depending on the weight of the subject to whom it is administered. For example, the supplement of the present invention can be prepared so that a dose of 1 mg/kg body weight/day or more, preferably 1 to 100 mg/kg body weight/day of the inorganic compound can be administered to a subject receiving the supplement of the present invention. Typically, the supplement of the present invention is administered 1 to 3 times per day.

Examples of the subjects who ingest the supplement of the present invention include humans, ruminants such as cattle, sheep, and goats, monogastric animals such as horses, pigs, chickens, dogs, and fish.

### [Feed]

The sporulation inhibitor of the present invention can be given to nonhuman animals such as livestock as it is, or can be used as feed together with excipients or diluents such as corn, soy flour, rice bran, fish meal, and beer yeast.

The feed of the present invention may also contain an optional additive that can be contained in conventional feeds. It is appropriate to take the feed of the present invention continuously every day. The amount of the feed to be taken varies depending on the size of the livestock. For example, in the case of chickens, the amount of the sporulation inhibitor to be taken per day is such that the inorganic compound is about 0.013 to 2.7 mmol/kg (1 to 340 ppm), preferably 0.14 to 1.4 mmol/kg (10 to 170 ppm), with respect to the conventional feed other than the sporulation inhibitor. Further, in the case of the sporulation inhibitor containing a polysaccharide is included in a conventional feed, the amount to be taken increases accordingly. For example, when containing 133 ppm of arabic gum as a polysaccharide, the sporulation inhibitor is desirably given such that the inorganic compound is about 0.013 to 1.4 mmol/kg (1 to 170 ppm), preferably 0.14 to 0.7 mmol/kg (10 to 85 ppm), further preferably 0.54 to 0.8 mmol/kg (40 to 60 ppm), with respect to the conventional feed. In this description, "ppm" means "ppm by mass".

In this description, the "livestock" means living things fed by humans. Specifically, ruminants such as cattle, sheep, and goats, and monogastric animals such as horses, pigs, chickens, dogs, and fish are included. The feed of the present invention is particularly preferably given to monogastric animals.

The feeding method with the sporulation inhibitor of the present invention is not particularly limited.

### Examples

### Example 1. Bacterial cell settling experiment

Clostridium perfringens ATCC10873 was cultured on a GAM plate (modified gam broth "NISSUI", available from Nissui Pharmaceutical Co., Ltd.) at 37°C under anaerobic conditions for 24 hours. The anaerobic culture was performed using an anaerobic culture kit "AnaeroPack", available from MITSUBISHI GAS CHEMICAL COMPANY, INC.

The culture obtained was suspended in 4 mL of pure water produced using a pure water production device, available from Merck Millipore, and the optical density (OD) of the suspension was adjusted to about 2.0 at a wavelength of 660 nm.

The settling promoters described in Table 1 each were added at a concentration shown in Table 1 to a vial container containing 0.5 mL of the suspension, and each substance was brought into contact with Clostridium perfringens. Arabic Gum ("AG") was used as a positive control.

The vial container was left standing at 37°C for 8 hours. During this time, the optical density of the sample was automatically and continuously measured.

### <Evaluation>

When a decrease in optical density of a sample 8 hours after the settling promoter was added was twice or more a decrease in optical density of the sample in 8 hours with no addition of the settling promoter (that is, a decrease in optical density due to natural settling of bacterial cells), the settling promoter was determined to have a settling effect.

### <Results and discussion>

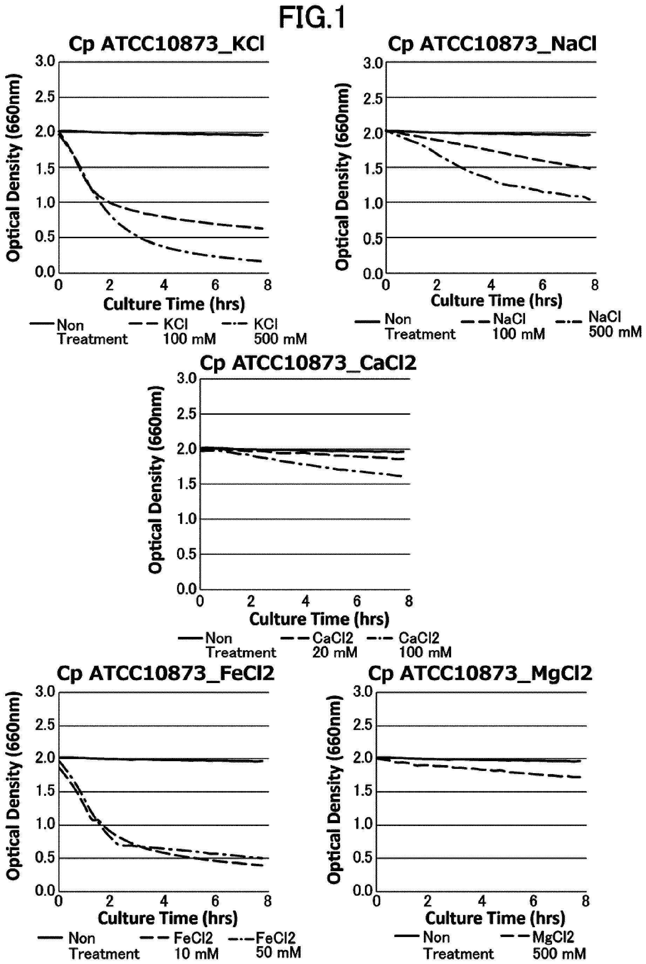

Table 1 and Figure 1 show the results. AG, which exerted a settling effect on Gram-negative bacteria, did not exert a settling effect on C. perfringens that is a Gram-positive bacterium. In the absence of AG, settling was confirmed at KC1 100 mM or more, NaCl 500 mM, or FeCl₂ 10 mM or more. This is considered because the cations reduce the repulsive force by changing the surface potential of Clostridium perfringens bacterial cells. In the absence of AG, MgCl₂ (500 mM), which did not exert a settling effect, exerted a settling effect when used in combination with AG.

**[Table 1]**

| **Table 1: Settling experiment of Clostridium perfringens ATCC10873** | | | |
|---|---|---|---|
| **Settling promoter** | Conc(mM) | w/AG | Settling |
| **NT** | - | - | Poor |
| **AG** | - | 1% | Poor |
| **KCl** | 100 | - | Good |
| | 500 | - | Good |
| | 500 | 1% | Good |
| **NaCl** | 20 | - | Poor |
| | 100 | - | Poor |
| | 500 | - | Good |
| | 500 | 1% | Good |
| **MgCl2** | 500 | - | Poor |
| | 500 | 1% | Good |
| **CaCl2** | 20 | - | Poor |
| | 100 | - | Poor |
| **FeCl2** | 10 | - | Good |
| | 50 | - | Good |

| | | | |
|---|---|---|---|
| "-": Additive-free "Good": Settling effect observed "Poor": No settling effect observed | | | |

### Example 2. Agglomeration and bacterial cell shape observation experiment

The same experiment as in Example 1 was conducted using three Clostridium perfringens strains [ATCC10873 (toxin producing Type A), SM101 (toxin producing Type F: CPE toxin producer), and CNEOP004 (toxin producing Type G: netB toxin producer), all bacilli]. However, the optical density of each sample was not measured.

The sample was collected 8 hours from the addition of the settling promoter, and the presence or absence of agglomeration of each bacterial strain and the shape of bacterial cells were observed using an upright microscope (model: BX50, available from OLYMPUS CORPORATION).

### <Results>

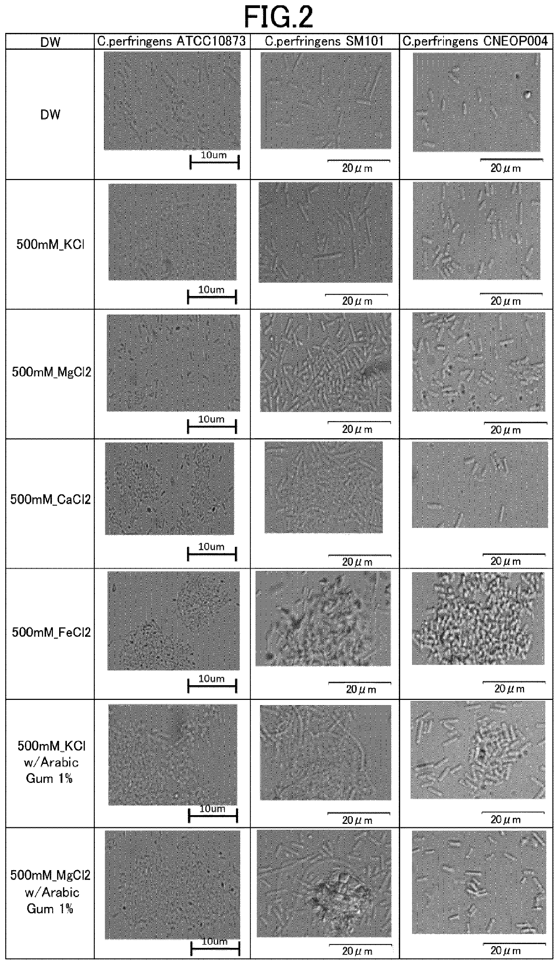

Table 2 and Figure 2 show the results.

AG, which exerted an agglomeration effect on Gram-negative bacteria, did not exert an agglomeration effect on Clostridium perfringens. When KC 1 or NaCl was added to the sample of C. perfringens in the absence of AG, the shape of the bacterial cells changed from bacilli to cocci, but the bacterial cells themselves did not agglomerate. In the coexistence of KC1 or NaCl with AG, the bacterial cells agglomerated in the state of cocci.

When MgC1₂ was added to the sample in the absence of AG, the shape as bacilli remained unchanged, and no agglomeration was observed, but the agglomeration of Clostridium perfringens was observed in the coexistence of AG with MgC1₂.

When CaC1₂ and FeC1₂ each were added to the sample in the absence of AG, Clostridium perfringens agglomerated in the state of bacilli.

**[Table 2]**

| **Table 2: Observation experiment of agglomeration and cell shapes of Clostridium perfringens** | | | | |
|---|---|---|---|---|
| **Table 2-1: Clostridium perfringens ATCC10873** | | | | |
| **Settling** promoter | Conc(mM) | w/ AG | Agglomeration | Shape |
| **NT** | - | - | Poor | Rod-shaped |
| **KCl** | 100 | - | Poor | Spheroidal |
| | 500 | - | Poor | Spheroidal |
| | 500 | 1% | Good | Spheroidal |
| **NaCl** | 500 | - | Poor | Spheroidal |
| | 500 | 1% | Good | Spheroidal |
| **MgCl2** | 500 | - | Poor | Rod-shaped |
| | 500 | 1% | Good | Rod-shaped |
| **CaCl2** | 20 | - | Good | Rod-shaped |
| | 100 | - | Good | Rod-shaped |
| **FeCl2** | 10 | - | Good | Rod-shaped |
| | 50 | - | Good | Rod-shaped |

| **Table 2-2 Clostridium perfringens SM101 (CPE toxin producer)** | | | | |
|---|---|---|---|---|
| **Settling promoter** | Conc(mM) | w/ AG | Agglomeration | Shape |
| **NT** | - | - | - | Rod-shaped |
| **KCl** | 500 | - | Poor | Spheroidal |
| | 500 | 1% | Good | Spheroidal |
| **MgCl2** | 500 | - | Poor | Rod-shaped |
| | 500 | 1% | Good | Rod-shaped |
| **CaCl2** | 500 | - | Good | Rod-shaped |
| **FeCl2** | 500 | - | Good | Rod-shaped |

| **Table 2-3 Clostridium perfringens CNEOP004 (netB toxin producer)** | | | | |
|---|---|---|---|---|
| **Settling promoter** | Conc(mM) | w/ AG | Agglomeration | Shape |
| **NT** | - | - | - | Rod-shaped |
| **KCl** | 500 | - | Poor | Spheroidal |
| | 500 | 1% | Good | Spheroidal |
| **MgCl2** | 500 | - | Poor | Rod-shaped |
| | 500 | 1% | Good | Rod-shaped |
| **CaCl2** | 500 | - | Good | Rod-shaped |
| **FeCl2** | 500 | - | Good | Rod-shaped |

| | | | | |
|---|---|---|---|---|
| "-": Additive-free "Good": Agglomeration effect observed "Poor": No agglomeration effect observed | | | | |

### Example 3. Measurement of surface potential of Clostridium perfringens bacterial cells

An experiment was conducted under the same conditions as in Example 1 using three Clostridium perfringens strains (ATCC10873, SM101, and CNEOP004). However, the optical density of each sample was not measured.

The sample was collected 8 hours from the addition of the settling promoter, diluted 100 times, and then the surface potential of the Clostridium perfringens bacterial cells was measured using Zetasizer Nano (model number: Nano-ZS), available from Malvern Panalytical. The surface potential of the settling promoter before its addition and the surface potential of the settling promoter were also measured (the concentration at the time of measurement was adjusted to the concentration of the sample at the time of measuring the surface potential).

### <Results and discussion>

Table 3 and Table A show the results. Clostridium perfringens did not agglomerate when Arabic Gum or KCl was added alone, but it agglomerated when both were added together. This is probably because the surface layer of the bacterial cells was negatively charged in water, but the negative charge on the surface potential of the bacterial cells increased by addition of KC1, the surface potential became neutral by the coexistence of Arabic Gum, and the bacterial cells and KCl mutually interfered with Arabic Gum, resulting in agglomeration (Table 3). In the case of ATCC10873 strain, when MgC1₂ was added, the surface potential of the bacterial cells was once neutral, and then the surface potential of the bacterial cells changed again (negatively charged) by the addition of Arabic Gum (Table 3-1). This is probably because the bacterial cells and MgC1₂ mutually interfered with Arabic Gum and agglomerated. On the other hand, it is considered that, in SM101 and CNEOP004, the surface potential of the bacterial cells significantly changed toward the neutral side when adding MgC1₂ and agglomerated (Table 3-2, Table 3-3). It is considered that, when adding each of CaC1₂ (20 mM or more) and FeC1₂ (10 mM or more), the surface potential of the bacterial cells significantly changed toward the neutral side in the absence of AG, and the bacterial cells mutually interfered with each of CaC1₂ and FeC1₂ and agglomerated (Table 3).

**[Table 3]**

| **Table 3: Measurement of surface potential of Clostridium perfringens bacterial cells** | | | | |
|---|---|---|---|---|
| Table 3-1: Clostridium perfringens ATCC10873 | | | | |
| Settling promoter | Conc(mM) | w/ AG | Agglomeration | Zeta potential |
| NT | - | - | Poor | -16.1 |
| KCl | 500 | - | Poor | -29.0 |
| | 500 | 1% | Good | -10.2 |
| MgCl2 | 500 | - | Poor | -3.2 |
| | 500 | 1% | Good | -12.9 |
| CaCl2 | 20 | - | Good | -4.4 |
| | 100 | - | Good | -4.0 |
| FeCl2 | 10 | - | Good | 0.8 |
| | 50 | - | Good | 20.7 |

| Table 3-2 Clostridium perfringens SM101 | | | | |
|---|---|---|---|---|
| Settling promoter | Conc(mM) | w/ AG | Agglomeration | Zeta potential |
| NT | - | - | - | -41.1 |
| KCl | 500 | - | Poor | -30.2 |
| | 500 | 1% | Good | -26.5 |
| MgCl2 | 500 | - | Good | -12.1 |
| | 500 | 1% | Good | -10.2 |
| CaCl2 | 500 | - | Good | -14.3 |
| FeCl2 | 500 | - | Good | 4.1 |

| Table 3-3 Clostridium perfringens CNEOP004 | | | | |
|---|---|---|---|---|
| Settling promoter | Conc(mM) | w/ AG | Agglomeration | Zeta potential |
| NT | - | - | - | -34.6 |
| KCl | 500 | - | Poor | -25 |
| | 500 | 1% | Good | -23 |
| MgCl2 | 500 | - | Good | -12 |
| | 500 | 1% | Good | -11.8 |
| CaCl2 | 500 | - | Good | -12.3 |
| FeCl2 | 500 | - | Good | 21.5 |

| | | | | |
|---|---|---|---|---|
| "-": Additive-free "Good": Agglomeration effect observed "Poor": No agglomeration effect observed | | | | |

**[Table A]**

| Table A: Zeta potential of substance itself | | |
|---|---|---|
| | Conc | Zeta potential |
| Clostridium perfringens ATCC10873 | - | -16.1 |
| Clostridium perfringens SM101 | - | -41.1 |
| Clostridium perfringens CNEOP004 | - | -34.6 |
| KCl | 0.5M | -4.3 |
| MgCl2 | 0.5M | 37.7 |
| CaCl2 | 0.5M | -1.8 |
| FeCl2 | 0.5M | 20.8 |
| Arabic Gum | 1% | -19 |

### Example 4. Phase-contrast microscopic observation of Clostridium perfringens cocci after KCl addition

In general, there are many reports that E.coli, Campylobacter, Helicobacter pylori change their shape to coccoid forms called "coccoid-forms" under stress environments, and Clostridium has also reported to become spheroidal (International Journal of Current Microbiology and Applied Sciences (2016), 5 (7), 210-223). The sporulation process requires cell division, which requires nutrients for the cell division. It has been reported that the spores of Clostridium perfringens, like Clostridium difficile, become vegetative cells after transitioning from Phase-bright spores to Phase-dark spores (J. Am. Chem. Soc. (2014) v 136 pp 14498-14504).

In Example 2, since pure water containing no nutrients was used, the cocci observed in Example 2 were expected to be in "coccoid-form" rather than spores.

Therefore, it was investigated whether the cocci (diameter: 1 µm) produced by addition of KCl (0.5 M) to Clostridium perfringens were vegetative cells or spores. Since the water content decreases, and DNA or the like is concentrated, spores glow white under a phase-contrast microscope (Phase-bright spores). Phase-dark spores are seen before germination into vegetative cells, their color is dark when viewed with a phase-contrast microscope. Vegetative cells have a pale color and black spots observed in cells under a phase-contrast microscope.

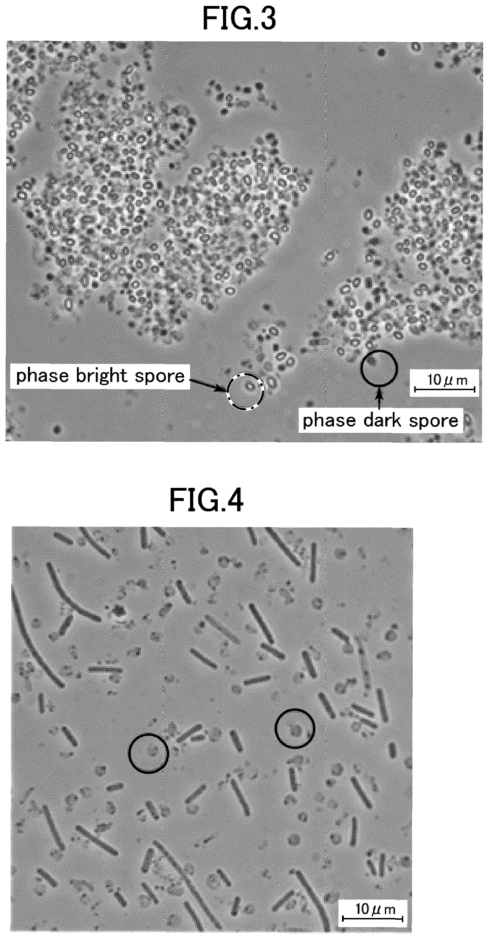

As a reference, spores of Clostridium perfringens strain SM101 were observed with a phase-contrast microscope (Figure 3). In Figure 3, Phase-bright spores are oval-shaped that glow white, and Phase-dark spores are the same shape but appear darker. In addition, fragments of vegetative bacteria were observed.

On the other hand, coccoid bacteria produced by addition of KCl (500 mM) to Clostridium perfringens strain SM101 were observed with a phase-contrast microscope in Example 2 (Figure 4), and since they had a pale color and black spots observed in cells like bacilli, which are vegetative cells, it was presumed that they were not spores.

Accordingly, it is considered that the shape change of Clostridium perfringens bacterial cells due to the addition of KCl or the like occurred in the state of vegetative cells without forming spores.

### Example 5. Agglomeration observation experiment of enteric bacteria

For enteric bacteria other than Clostridium perfringens, agglomeration of each bacterial strain was observed in the same manner as in Example 2. As enteric bacteria, Lactobacillus casei ATCC393 and Bifidobacterium animalis JCM1190, which are Gram-positive bacteria, and Salmonella enterica IAM1648 and E.coli MG1655, which are Gram-negative bacteria, were selected. Culturing at 37°C was performed for Lactobacillus casei ATCC393 and Bifidobacterium animalis JCM1190 on an MRS plate (available from Difco Laboratories), E.coli MG1655 on an LB plate (available from Becton, Dickinson and Company), and Salmonella enterica IAM1648 on an NB plate (available from Difco Laboratories). Bifidobacterium animalis JCM1190, like Clostridium perfringens, was cultured under anaerobic conditions using an anaerobic culture kit "AnaeroPack", available from MITSUBISHI GAS CHEMICAL COMPANY, INC. Other bacteria were cultured under aerobic conditions.

Table 4 shows the results. For reference, the results for Clostridium perfringens ATCC10873 conducted in Example 2 are also described together.

The agglomeration effect of KCl in coexistence with Arabic Gum was specific to Clostridium perfringens among Gram-positive bacteria and was not exerted on Lactobacillus casei and Bifidobacterium animalis. Gram-negative bacteria Salmonella enterica and E.coli agglomerated by Arabic Gum. These results agreed with the results reported in WO2019/177172. All the Gram-negative bacteria did not agglomerate by KCl alone but agglomerated when used in combination with Arabic Gum. Therefore, it was suggested that use of KC1 and Arabic Gum in combination specifically agglomerated Clostridium perfringens, Salmonella enterica, and E.coli, which are called bad bacteria.

In addition, FeCl₂ showed an agglomeration effect on Clostridium perfringens, Lactobacillus casei, and Bifidobacterium animals, which are Gram-positive bacteria.

**[Table 4]**

| **Table 4: Agglomeration observation of intestinal microorganisms** | | | | | | | |
|---|---|---|---|---|---|---|---|
| Settling promoter | Conc(mM) | w/ AG | Clostridium perfringens ATCC10873 | Lactobacillus casei ATCC393 | Bifidobacterium animalis JCM1190 | Salmonella enterica IAM1648 | Escherichia coli MG1655 |
| NT (additive-free) | - | - | Poor | Poor | Poor | Poor | Poor |
| Arabic Gum (AG) | - | 1% | Poor | Poor | Poor | Good | Good |
| KCl | 500 | - | Poor | Poor | Poor | Poor | Poor |
| | 500 | 1% | Good | Poor | Poor | Good | Good |
| MgCl2 | 500 | - | Poor | Poor | Poor | Poor | Poor |
| | 500 | 1% | Good | Good | Poor | Good | Good |
| CaCl2 | 500 | - | Good | Poor | Poor | Poor | Poor |
| FeCl2 | 500 | - | Good | Good | Good | Poor | Poor |

| | | | | | | | |
|---|---|---|---|---|---|---|---|
| "Good": Those in which agglomeration observed under upright microscope, "Poor": Those in which no agglomeration observed under upright microscope "-": Additive-free | | | | | | | |

### Example 6. Agglomeration observation experiment of three types of enteric bad bacteria

In the same manner as in Example 5, three strains of Clostridium perfringens ATCC10873, Salmonella enterica IAM1648, and E.coli MG1655 were cultured. A suspension of each bacterial strain was prepared in the same manner as in Example 1. After being adjusted so that the optical density (OD) of each suspension at a wavelength of 660 nm was about 0.6 to 0.7, the suspensions of the three bacterial cells were mixed, and the mixed bacterial cells were collected using a centrifuge and adjusted so that the final optical density was about 2.0 (at a wavelength of 660 nm) in a vial container. A mixed solution of KCl 500 mM and Arabic Gum 1% and a mixed solution of NaCl 500 mM and Arabic Gum 1% were each added to the vial container to be brought into contact with the aforementioned mixed bacterial cells. Each vial container was left standing at 37°C, and the optical density was automatically and continuously measured.

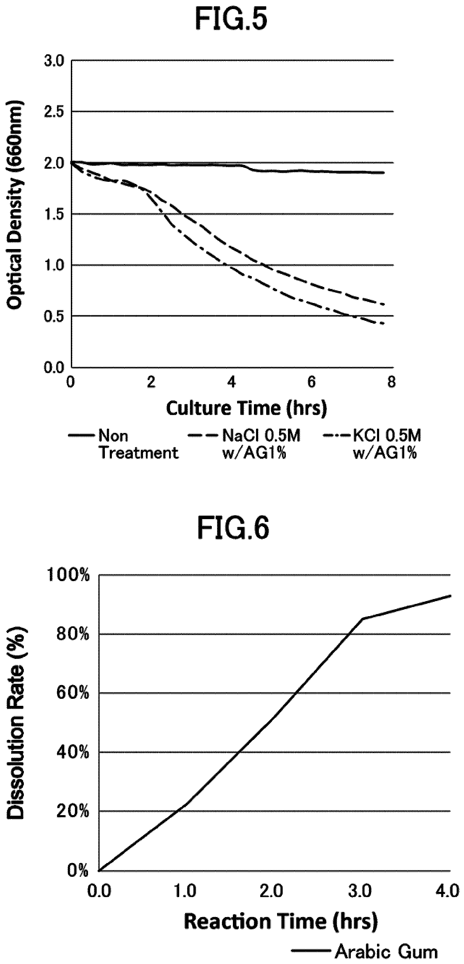

Figure 5 shows the results. It was confirmed that the combination of KCl 500 mM and Arabic Gum 1% and the combination of NaCl 500 mM and Arabic Gum 1% can quickly agglomerate the three types of enteric bad bacteria.

### Example 7. Preparation of coated feed sporulation inhibitor

Arabic Gum (available from Wako Pure Chemical Industries, Ltd.) and KCl (available from Wako Pure Chemical Industries, Ltd.) were used as core materials, and hydrogenated rapeseed oil (melting point: 67°C) and Benzoin Resin (available from Chuo-Koryo Inc.) were used as coating agents. A coated feed sporulation inhibitor was obtained by spraying a predetermined amount of a coating agent liquified by heating to a temperature higher than the melting point onto a powdered or granulated core.

Arabic Gum was coated in one layer, and 75 parts by mass of the core material was coated with 25 parts by mass of hydrogenated rapeseed oil. Hereinafter, this additive will be referred to as "Coated-Arabic Gum". On the other hand, KCl was coated in two layers, and 84.77 parts by mass of the core material was coated with 2.23 parts by mass of Benzoin Resin as the first layer (inside layer) and with 13 parts by mass of hydrogenated rapeseed oil as the second layer (the outer layer). Hereinafter, this additive will be referred to as "Coated-KCl".

### Example 8. Enteric test of Coated-Arabic Gum

### (Artificial gastric juice treatment)

0.2% NaCl and 0.2% pepsin (from Porcine stomach Mucosa, 1:5,000, 2,500 units/mg) were added to pure water produced using a pure water production device, available from Merck Millipore, and after the pH was adjusted to 2, Coated-Arabic Gum prepared in Example 7 was added, and it was subjected to an enzymatic treatment at 37°C for 2 hours. Enteric properties were evaluated by automatically and continuously measuring the optical density during this period. "2 hours" assumes the time from when the feed reaches the chicken's stomach until it passes through it.

### (Artificial intestinal juice treatment)

After artificial gastric juice treatment, 0.2% trypsin (from Porcine Pancreas, 1:5,000; 4,500 units/mg) was added, after the pH was adjusted to 6, it was subjected to an enzymatic treatment at 37°C for 2 hours. Enteric properties were evaluated by automatically and continuously measuring the optical density during this period. "2 hours" assumes the time from when the feed reaches the chicken's intestine until it passes through it.

For the optical density in both treatments, turbidity (Optical density [OD] at a wavelength of 190 nm) was measured using a spectrophotometer UVmini-1240, available from SHIMADZU CORPORATION. In addition, hydrochloric acid and sodium hydroxide were used as pH adjusters in both treatments.

Figure 6 shows the results. While the dissolution rate within 2 hours from the start of gastric juice treatment was suppressed to about 60% or less, it exceeded 90% in intestinal juice treatment. From these results, it was found that good release control could be achieved in the sample that used hydrogenated rapeseed oil as a coating agent.

### Example 9. Coated-KCl dissolution test

Coated-KCl prepared in Example 7 was added to pure water produced using a pure water production device, available from Merck Millipore, and a dissolution test was conducted at 37°C. The dissolution rate was measured by automatically and continuously measuring the optical density during this period. For the optical density in both treatments, turbidity (Optical density [OD] at a wavelength of 190 nm) was measured using a spectrophotometer UVmini-1240, available from SHIMADZU CORPORATION.

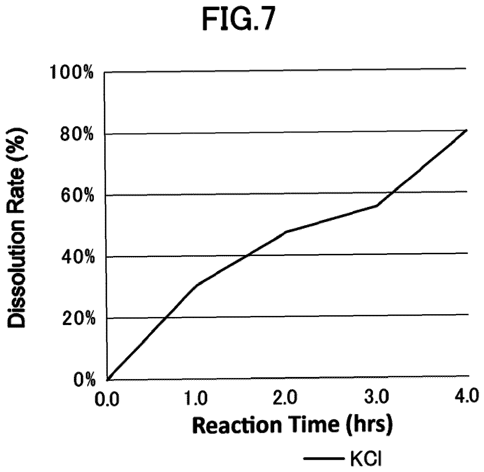

Figure 7 shows the results. While the dissolution rate within 2 hours from the start was suppressed to about 50% or less, it reached 80% in intestinal juice treatment. From these results, it was found that good release control could be achieved in the sample that used a two-layer coating of Benzoin Resin and hydrogenated rapeseed oil as coating agents.

### Example 10. Clostridium perfringens infection test

Coated-KCl prepared in Example 7 was added to the feed described in Hofacre, C.L., et al., Avian Dis. 1998; 42 (3): 579-84 so that the amount in the core material was 40 ppm, to obtain a feed composition. The experiment was outsourced to SPRG (Strategic Public Relations Group) and was performed in the United States.

In a cage-free chicken house, 25 first born chick broilers were fed with the feed composition in one area, and a total of 500 chickens in 20 replicates were bred for 6 weeks from the time of feeding. For the test conditions, a commercially used coccidia attenuated vaccine (0.007 mL vaccine/bird) was administered on Day 0. Thereafter, after 4 hours of fasting and 2 to 3 hours of water deprivation, Clostiridium perfringens was added to the drinker at 1 × 10⁸ cfu/mL, and water was supplied on Days 14, 15 and 16.

Necrotic enteritis (NE)-induced mortality and NE-induced intestinal damage score during the breeding period were evaluated. The NE-induced intestinal damage score was evaluated as 0 = none, 1 = mild, 2 = moderate, and 3 = severe, and the average of the scores was calculated.

The additive-free area was used as a control ("Challenge Control" in Table 5). As a positive control, bacitracin methylene disalicylate (BMD) was used. BMD is known as a substance having an effect against necrotic enteritis caused by Clostridium perfringens and is widely used as an antibacterial growth promoter (AGP), which is one of AGPs used the most in the United States. BMD was added to the feed described in the aforementioned Hofacre literature at 55 ppm by volume, to create the feed composition as a positive control. As BMD, a commercially available product (BMD (R) of Zoetis Inc., uncoated) was used as it was.

Table 5 shows the results. BMD reduced mortality to 13.2% as compared with Challenge Control. Coated-KCl reduced mortality to 3.8% as compared with Challenge Control. N.E. lesion score significantly improved the score as compared with Challenge Control (Control: 0.75, BMD: 0.30, Coated-KCl: 0.52).

**[Table 5]**

| **Table 5: Clostridium perfringens infection test** | | | | |
|---|---|---|---|---|
| Treatment | Dead birds | Day 0-14 | Day 15-28 | Day 29-42 |
| Challenge Control | Total | 12 | 72 | 12 |
| | NE Caused | 0 | 69 | 1 |
| BMD (55ppm) | Total | 19 | 10 | 6 |
| | NE Caused | 0 | 4 | 0 |
| Coated-KCl (47ppm) | Total | 15 | 63 | 6 |
| | NE Caused | 0 | 51 | 0 |

| Treatment | NE Caused Mortality | NE Lesion Score | | |
|---|---|---|---|---|
| Challenge Control | 14.00 | 0.75 | | |
| BMD (55ppm) | 0.80 | 0.30 | | |
| Coated-KCl (47ppm) | 10.20 | 0.52 | | |

| | | | | |
|---|---|---|---|---|
| Clinical NE Results: The number indicates the average. One-way ANOVA with post-hoc Tukey's test was done to compare the treatments. NE Lesion Score: Randomly select three (3) birds per pen and scored on day 17. (0=none, 1=mild, 2= moderate, 3=severe) | | | | |

### Example 11. Weight gain effect test

Coated-KCl and Coated-Arabic Gum prepared in Example 7 were added to the basic feed without feed additives in Table 6, so that the amounts of the core material were 40 ppm by volume and 100 ppm by volume respectively, to obtain feed compositions.

**[Table 6]**

| **Table 6: Feed composition** | |
|---|---|
| Raw material | Mixing ratio, mass% |
| Corn | 45.4 |
| Grain sorghum | 10 |
| Soybean meal | 30 |
| Corn gluten meal | 4 |
| Fishmeal (CP 65%) | 3 |
| L-Lysine hydrochloride | 0.31 |
| DL-Methionine | 0.35 |
| L-Threonine | 0.12 |
| L-Arginine | 0.16 |
| Animal fats and oils | 3.49 |
| Dibasic calcium phosphate | 1.45 |
| Calcium carbonate | 1.06 |
| Salt | 0.3 |
| Vitamin/mineral premix | 0.25 |
| Choline chloride | 0.02 |
| L-Valine | 0.07 |
| Total | 100 |

The test chicks were first born male chicks of a breed exclusively for broilers (UK Chunky) weighing 38 to 46 g and were selected for use in the test. In a cage-free chicken house, all test groups were fed with the basic feed shown in Table 6 without feed additives, and 50 test chicks per group with approximately equal weight distribution and 3 replicates each were bred for 3 weeks from the time of feeding. As the test conditions, in order to impose stress on the environmental conditions, they were bred on a wet floor by spreading about 1 L of water per group once a day during the breeding period the entire surface of the floor. The test areas were 1) Control (Non treatment), 2) Positive control (BMD 55 ppm), 3) Coated-Arabic Gum (133 ppm), and 4) Coated-Arabic Gum (133 ppm) + Coated-KCl (47 ppm). During the breeding period, the weight gain effect (Body Weight Gain; BWG) of birds was evaluated. Table 7 shows the results. The results were shown with a negative control as 100.

For BMD, weight gain was improved in all areas (0 to 21 days old) as compared with Control (Non treatment). On the other hand, although no significant difference was observed in the area combining Coated-Arabic Gum and Coated-KCl as compared with Control, the weight gain effect became apparent with growth, and the weight gain effect was observed in the area (15 to 21 days old). In general, the shipment was at 6 to 7 weeks old, and thus a sufficient weight gain effect was expected.

**[Table 7]**

| **Table 7 Weight gain effect test** | | | |
|---|---|---|---|
| Treatment | Day 0-7 | Day 8-14 | Day 15-21 |
| Control (non treatment) | 100.0 | 100.0 | 100.0 |
| BMD (55ppm) | 106.0 | 103.9 | 103.5 |
| Coated Arabic Gum(133ppm) | 97.9 | 102.9 | 98.0 |
| Coated Arabic Gum(133ppm) w/ Coated-KCl (47ppm) | 98.7 | 100.2 | 102.1 |

| | | | |
|---|---|---|---|
| BWG % : versus Control (Non treatment) | | | |

## Claims

1. A sporulation inhibitor of Clostridium perfringens comprising at least one inorganic compound at a concentration that allows Clostridium perfringens to settle in vivo, wherein the inorganic compound is at least one salt selected from the group consisting of K, Na, Mg, Ca, and Fe, and the inorganic compound is coated with a protective layer containing a hydrogenated vegetable oil.

2. The sporulation inhibitor according to claim 1, wherein
the inorganic compound is a salt with an anion formed from an acid selected from the group consisting of hydrochloric acid, sulfuric acid, phosphoric acid, carbonic acid, lignosulfonic acid, silicic acid, lactic acid, citric acid, gluconic acid, succinic acid, fumaric acid, iodine, and iodic acid.

3. The sporulation inhibitor according to claim 1 or 2, wherein
the inorganic compound is at least one selected from the group consisting of KCl, NaCl, MgCl₂, CaCl₂, and FeCl₂.

4. The sporulation inhibitor according to any one of claims 1 to 3, wherein
the inorganic compound is KCl.

5. The sporulation inhibitor according to any one of claims 1 to 4, further comprising a polysaccharide.

6. The sporulation inhibitor according to claim 5, wherein
the polysaccharide is at least one selected from the group consisting of pullulan, xanthan gum, guar gum, carrageenan, arabic gum, pectin, carboxymethylcellulose, chondroitin, tara gum, locust bean gum, alginates (such as sodium salt, potassium salt, calcium salt, or ammonium salt), alginic acid ester, and a mixture thereof.

7. The sporulation inhibitor according to claim 5, wherein
the polysaccharide is arabic gum.

8. The sporulation inhibitor according to any one of claims 1 to 7, wherein
the inorganic compound and the polysaccharide contained as needed are coated with a protective layer containing a hydrogenated vegetable oil.

9. The sporulation inhibitor according to claim 8, wherein
the hydrogenated vegetable oil is a hydrogenated oil of rapeseed oil, linseed oil, safflower oil, sunflower oil, soybean oil, corn oil, peanut oil, cottonseed oil, sesame oil, rice oil, olive oil, palm oil, palm kernel oil, or coconut oil.

10. The sporulation inhibitor according to any one of claims 1 to 7, wherein
the inorganic compound and the polysaccharide contained as needed are protected by two layers of hydrogenated rapeseed oil and benzoic acid resin.

11. A supplement to prevent or treat Clostridium perfringens infection, comprising:
the sporulation inhibitor according to any one of claims 1 to 10.

12. The supplement according to claim 10 or 11, comprising:
an inorganic compound at a concentration that allows administration of the inorganic compound at a dose of 1 mg/kg body weight/day or more.

13. The supplement according to claim 10, wherein
the Clostridium perfringens infection is necrotic enteritis.

14. A feed comprising:
the sporulation inhibitor according to any one of claims 1 to 8.

15. The feed according to claim 14, further comprising a conventional feed, wherein
the concentration of the inorganic compound is 0.013 mmol/kg or more with reference to the mass of the conventional feed.

16. A method for preventing or treating Clostridium perfringens infection of nonhuman animals, the method comprising:
administering an inorganic compound of at least one salt selected from the group consisting of K, Na, Mg, Ca, and Fe to the intestinal tract of a nonhuman animal.

17. A method for inhibiting sporulation of Clostridium perfringens in vitro, the method comprising incubating at an effective concentration of 500 mM or more of an inorganic compound of at least one salt selected from the group consisting of K, Na, Ca, and Fe.
